# EUROPÄISCHE PATENTANMELDUNG

(11) **EP 1 088 807 A1**
(43) Veröffentlichungstag der Anmeldung: **04.04.2001**
(21) Anmeldenummer: 00117401.0
(22) Anmeldetag: 11.08.2000
(51) Int. Cl.: C07C 37/08, C07C 45/53

(54) **Verfahren zur Herstellung von Phenol, Methylethylketon und Aceton**

(30) Priorität: 30.09.1999 DE 19946887
(71) Anmelder: Phenolchemie GmbH & Co. KG, 45966 Gladbeck (DE)
(72) Erfinder: Pompetzki, Werner, Dr., 46284 Dorsten (DE); Gerlich, Otto, Dr., 45966 Gladbech (DE); Kleinloh, Werner, Dr., 45721 Haltern (DE)
(74) Vertreter: Olbricht, Gerhard, Dr.

(57) **Zusammenfassung**

Bei der Herstellung von Phenol, Methylethylketon und Aceton durch Hocksche Spaltung ausgehend von Gemischen, die neben sek.-Butylbenzol 30 bis 70 Gew.-% Cumol (bezogen auf sek.-Butylbenzol) enthalten, fallen in nennenswertem Umfang Rückstände an, die lediglich thermisch verwertet werden können.

Durch die erfindungsgemäße Begrenzung des Cumolgehalts in den sek.-Butylbenzol/Cumol-Gemischen auf 3 bis 15 Gew.-% wird eine Veränderung des Nebenproduktspektrums hin zu verwertbaren Substanzen und eine Ausbeuteverbesserung an Wertprodukten insgesamt erzielt.

Herstellung von Phenol, Methylethylketon, Aceton und Acetophenon.

## Beschreibung

Die vorliegende Erfindung betrifft ein Verfahren zur Herstellung von Phenol, Methylethylketon (kurz: MEK) und Aceton durch Hocksche Spaltung eines sek.-Butylbenzolhydroperoxid und Cumolhydroperoxid enthaltenden Gemisches.

Phenol ist ein wichtiger chemischer Grundstoff mit breiter Anwendungspalette. Neben seiner Verwendung als Lösemittel wird Phenol unter anderem zur Herstellung von Phenol-Harzen, Bisphenol-A, ε-Caprolactam, Adipinsäure, Alkylphenolen und Weichmachern eingesetzt.

Es ist bekannt, Phenol durch Hocksche Spaltung eines geeigneten Hydroperoxids herzustellen. Dabei entsteht neben dem Phenol als Hydroxy-Verbindung stets als Koppelprodukt eine Carbonyl-Verbindung, die aus wirtschaftlichen Gründen einer geeigneten Verwertung zugänglich sein muß.

Phenol wird heutzutage in der Hauptsache durch Hocksche Spaltung von Cumolhydroperoxid gewonnen, wobei als Koppelprodukt Aceton entsteht. Aceton besitzt ebenfalls vielfältige Anwendungsmöglichkeiten, nämlich z. B. als Lösemittel oder zur Herstellung von u. a. Methylmethacrylat.

Bei diesem sogenannten Cumolverfahren zur Herstellung von Phenol und Aceton wird zunächst Cumol mit vorzugsweise Luft oder Sauerstoff zu Cumolhydroperoxid oxidiert, welches - nach üblicherweise einer destillativen Aufkonzentrierung auf 60 bis 85 Gew.-% durch Abtrennen von nicht umgesetztem Cumol - anschließend unter Säurekatalyse mit vorzugsweise Schwefelsäure als Katalysator zu Phenol und Aceton gespalten wird. Einen guten Überblick über das Cumolverfahren geben z. B. Weissermel/Arpe, Industrielle Organische Chemie, 2. Auflage, Verlag Chemie, 1978, oder Ullmann's Encyclopedia of Industrial Chemistry, Vol. A 19, S. 302 ff., VCH Verlagsgesellschaft, 1991. Neuere Weiterentwicklungen des Cumolverfahrens betreffen vor allem den Bereich der Cumolhydroperoxid-Spaltung und die Aufarbeitung des Spaltprodukts zur Verringerung der Nebenproduktbildung und des Energieverbrauchs, vgl. z. B. EP-0 589 588 A1, EP-0 670 296 A1 oder WO 97/06 905.

Die Offenlegungsschrift EP-0 548 986 A1 lehrt ein Verfahren zur Herstellung von Phenol und Methylethylketon (kurz: MEK) als Koppelprodukt durch Oxidation von sek.-Butylbenzol zu sek. -Butylbenzolhydroperoxid und Hocksche Spaltung des sek.-Butylbenzolhydroperoxids zu Phenol und MEK. MEK ist neben Aceton eines der technisch wichtigsten Ketone, das vor allem als Lack- und Harzlösemittel verwendet wird. In EP-0 548 986 A1 wird vorgeschlagen, als Ausgangsmaterial ein sek.-Butylbenzol einzusetzen, das entweder im wesentlichen frei von Ethylhydroperoxid, Carbonsäuren und Phenol oder im wesentlichen frei von styrolischen Verbindungen oder im wesentlichen frei von Methylbenzylalkohol ist. Durch geeignete, auf diese Einsatzmaterialien abgestimmte Prozeßführungen mit speziellen zusätzlichen Behandlungsschritten wird vor allem eine verbesserte Abtrennung unerwunschter Nebenprodukte erzielt. Dadurch kann nicht umgesetztes sek.-Butylbenzol zum Oxidationsschritt rückgeführt werden, ohne die Oxidationsgeschwindigkeit negativ zu beeinflussen.

Auch die Offenlegungsschrift EP-0 578 194 A2 betrifft ein Verfahren zur Herstellung von Phenol und MEK aus sek.-Butylbenzol. Es wird ein Verfahren beschrieben, das im Anschluß an die üblichen Verfahrensschritte Oxidation, Aufkonzentrierung und Spaltung spezielle Aufbereitungsschritte, insbesondere eine Alkaliwäsche der durch .Destillation abgetrennten MEK-Fraktion, aufweist, wodurch insgesamt ein MEK höherer Reinheit erhalten werden soll.

Aus dem US-Patent 4 532 360 ist ein direktes, einstufiges Verfahren bekannt, das auch zur Herstellung von Phenol und MEK aus sek. -Butylbenzol verwendet werden kann. Dabei wird die Oxidation von sek.-Butylbenzol in Gegenwart von Bromwasserstoff oder Chlorwasserstoff sowie mindestens eines Additivs aus der Gruppe Ceriumoxid, Triphenylborat, Bortriphosphat und Wasser durchgeführt, wodurch die direkte Spaltung des sek.-Butylbenzolhydroperoxids zu Phenol und MEK eintritt.

Das Patentdokument JP-62-114 922 vom 26. Mai 1987 beschreibt ein Verfahren zur gleichzeitigen Herstellung von Phenol, Aceton und MEK durch Oxidation von sek.-Butylbenzol mit molekularen Sauerstoff enthaltendem Gas in Gegenwart von Cumol oder Cumolhydroperoxid. Nach JP-62-114 922 ist die Oxidation des sek.-Butylbenzols zu sek.-Butylbenzolhydroperoxid relativ langsam und kann durch den Zusatz von 5 bis 60 Gew.-%, vorzugsweise 5 bis 30 Gew.-%, eines Cumolhydroperoxid-Konzentrats, das 65 bis 85 Gew.-% Cumolhydroperoxid enthält, oder die Zugabe von über 50 Gew.-%, vorzugsweise 30 bis 70 Gew.-%, Cumol zum sek. -Butylbenzol, wobei sich die Angabe der Gew.-% beim Cumol auf den Gehalt an sek.-Butylbenzol bezieht, wesentlich beschleunigt werden. Ferner wird die Bildung des unerwünschten Nebenproduktes Acetophenon, das bei der Oxidation von sek.-Butylbenzol in größeren Mengen entsteht, verringert. Somit werden in kürzerer Zeit höhere Ausbeuten an den Wertprodukten Phenol, Aceton und MEK erreicht. Die Oxidation selbst erfolgt bei Temperaturen von 90 °C bis 145 °C und Drücken von 2 bis 21 bar absolut. Ein weiterer Vorteil des Verfahrens aus JP-62-114 922 besteht darin, daß die Abhängigkeit der Oxidationsgeschwindigkeit vom Isobutylbenzolgehalt in sek.-Butylbenzol vermindert wird.

Wie eigene Untersuchungen, die den Anstoß für die vorliegende Erfindung gaben, allerdings zeigten, werden bei diesem Verfahren bedingt durch die Cumol- bzw. Cumolhydroperoxidanteile im Reaktionsgemisch während der Oxidation erhebliche Mengen an Dimethylphenylcarbinol gebildet. Gleichzeitig entsteht durch Zersetzung eines gewissen Teils des bei der Oxidation gebildeten sek.-Butylbenzolhydroperoxids das 2-Phenylbutanol. Beide Alkohole werden bei der säurekatalysierten Spaltung der Hydroperoxide in die entsprechenden styrolischen Verbindungen α-Methylstyrol, Phenylbuten-2 und Phenylbuten-1 überführt, wobei hohe Konzentrationen dieser styrolischen Verbindungen zu einem erheblichen Rückstandsanfall fuhren. Dieser resultiert aus einem vermehrten Anfall von aus den styrolischen Verbindungen gebildeten Dimeren und Alkylphenolen, die bei der (Weiter-)Reaktion von α-Methylstyrol mit Phenol bzw. von Phenylbutenen mit Phenol gebildet werden. Das Verfahren nach JP-62-114 922 verbessert also durch die Zugabe der beschriebenen Anteile an Cumol oder Cumolhydroperoxid zum sek.-Butylbenzol die Oxidationsgeschwindigkeit und vermindert die Acetophenon-Bildung, doch entstehen dadurch vermehrt andere Nebenprodukte, die - anders als Acetophenon - in jedem Fall als Rückstand aufwendig entsorgt werden müssen. Im Gegensatz zu etwa dimeren styrolischen Verbindungen ist es beim konventionellen Cumolverfahren nämlich durchaus üblich, auch Acetophenon als Wertprodukt zu isolieren und zu vermarkten, vgl. z. B. US-4 559 110 oder Römpp Lexikon Chemie, 10. Auflage, Band 1, 1996 (Stichwort: Acetophenon). Ein erhöhter Acetophenongehalt ist daher anders als ein höherer Gehalt an dimeren styrolischen Verbindungen nicht zwangsläufig von Nachteil, obgleich alle diese Nebenprodukte die Ausbeute an Phenol, MEK und Aceton verringern und damit prinzipiell in möglichst geringem Umfang gebildet werden sollten.

Damit stellt sich die Aufgabe, ein Verfahren zur Herstellung von Phenol, MEK und Aceton durch Oxidation eines Cumol- und sek.-Butylbenzol enthaltenden Gemisches und Hocksche Spaltung von bei der Oxidation gebildetem Cumol- und sek.-Butylbenzolhydroperoxid bereitzustellen, das eine verbesserte Ausbeute an insgesamt verwertbaren Produkten aufweist.

Diese Aufgabe wird erfindungsgemäß gelöst gemäß Patentanspruch 1 durch ein Verfahren zur Herstellung von Phenol, MEK und Aceton durch Oxidation eines Cumol und sek.-Butylbenzol enthaltenden Gemisches mit molekularen Sauerstoff enthaltendem Gas und Hocksche Spaltung von bei der Oxidation gebildetem Cumol- und sek.-Butylbenzolhydroperoxid, das dadurch gekennzeichnet ist, daß der Cumolgehalt im Cumol und sek.-Butylbenzol enthaltenden Oxidationsgemisch 3 bis 15 Gew.-% beträgt. Vorzugsweise beträgt der Cumolgehalt in diesem Gemisch 3 bis 10 Gew.-%, besonders bevorzugt von 5 Gew.-% bis 10 Gew.-%. Vorzugsweise besteht das Gemisch nur aus Cumol und sek.-Butylbenzol.

Überraschenderweise wurde gefunden, daß bereits im Vergleich zu JP-62-114 922 deutlich geringere Zusätze gemäß dieser Erfindung von Cumol zu sek.-Butylbenzol ausreichen, um eine höhere Oxidationsgeschwindigkeit bei der sek.-Butylbenzoloxidation zu erzielen. Gleichzeitig wird durch geringere Cumolzusätze aber das Nebenproduktspektrum positiv beeinflußt. Wie bereits erwähnt, bilden sich nach eigenen Untersuchungen bei der Oxidation eines Cumol- und sek.-Butylbenzol enthaltenden Gemisches durch Zersetzung von gebildetem Cumolhydroperoxid nennenswerte Mengen an Dimethylphenylcarbinol, die für den Rückstandsanfall von entscheidener Bedeutung sind, da sie in der anschließenden säurekatalysierten Hydroperoxid-Spaltung zu nicht vermarktungsfähigen Folgeprodukten wie dimeren styrolischen Verbindungen und Alkylphenolen umgesetzt werden und damit reine Ausbeuteverluste darstellen, die allenfalls thermisch verwertet werden können. Durch die erfindungsgemäß verringerten Cumolzusätze bei der Oxidation wird aber - durch den folglich geringeren Cumolhydroperoxidgehalt - auch der Carbinolgehalt im Oxidat und damit als Folge auch der Anfall nicht marktfähiger Nebenprodukte, die entsorgt werden müssen, verringert. Erfindungsgemäß ist daher auch der Zusatz von Cumolhydroperoxid direkt zur Oxidationsreaktionsmischung nicht erwünscht. Infolge des verringerten Cumolgehalts kann verglichen mit den Reaktionsergebnissen aus JP-62-114 922 je nach Reaktionsbedingungen eine stärkere Acetophenonbildung auftreten. Diese kann in der Regel aber in Kauf genommen werden, da sich das Acetophenon auf dem Fachmann aus dem Cumolverfahren bekannte Weise bei der Aufarbeitung des Produktstroms aus der Hockschen Spaltung isolieren und gewinnbringend vermarkten läßt. Wird durch Anwendung des erfindungsgemäßen Verfahrens so viel Acetophenon gebildet, daß insgesamt die Ausbeute an Phenol, Aceton und MEK sinkt, wird bevorzugt auch das Acetophenon als Wertprodukt isoliert, so daß dann die Ausbeute an verwertbaren Produkten in jedem Fall steigt.

Acetophenon findet als hochsiedendes Lösemittel u. a. für Farben und Harze Verwendung; es dient ferner z. B. als Ausgangsstoff für Synthesen in der pharmazeutischen Industrie. Quellen für Acetophenon sind laut Römpp-Lexikon Chemie, 10. Auflage, Band 1, 1996, lediglich das Erhitzen von Benzol mit Acetylchlorid und Aluminium-chlorid sowie seine Bildung als Nebenprodukt im Cumolverfahren.

Das erfindungsgemäße Verfahren verbessert die Verfahrensbedingungen bei der Umsetzung Cumol und sek.-Butylbenzol enthaltender Gemische also derart, daß neben einer immer noch hohen Oxidationsgeschwindigkeit der Anfall nur noch thermisch verwertbarer Rückstände verringert wird.

Das erfindungsgemäß in der Oxidation eingesetzte Gemisch, das Cumol und sek.-Butylbenzol enthält, kann entweder in Lagertanks vorgehalten werden oder durch Vermischen aus getrennten Quellen erst vor dem Eintritt in die Oxidationsreaktoren gebildet werden. Es ist auch möglich, sek. -Butylbenzol oder Cumol enthaltende Stoffströme getrennt in die Oxidationsreaktoren einzudosieren und dort zu vermischen. Vorzugsweise werden nur Gemische aus Cumol und sek. -Butylbenzol jeweils hoher Reinheit eingesetzt. Als Oxidationsreaktoren dienen in der Regel die vom Cumolverfahren bekannten Blasensäulenreaktoren. Die Oxidation erfolgt analog zum Cumolverfahren vorzugsweise ohne Katalysator bei Temperaturen von 100 °C bis 140 °C und Drucken von 1 bis 20 bar absolut in Gegenwart von molekularen Sauerstoff enthaltendem Gas, bis in der Regel ein Gesamtgehalt von bis zu 30 Gew.-% an Peroxiden im Oxidat enthalten ist. Als molekularen Sauerstoff enthaltendes Gas dienen vorzugsweise Luft oder Sauerstoff. Die restlichen Bestandteile des Oxidats umfassen neben den Peroxiden überwiegend nicht umgesetztes sek.-Butylbenzol und Cumol, da vorzugsweise sek.-Butylbenzol/Cumol-Gemische hoher Reinheit eingesetzt werden. Ferner sind bei der Oxidation gebildete Nebenprodukte enthalten. Dazu zählen, wie erwähnt, insbesondere Dimethylphenylcarbinol, Acetophenon und 2-Phenylbutanol. Wie beim herkömmlichen Cumolverfahren kann der Oxidaistrom direkt oder über eine Vorlage als Zwischenspeicher einer Konzentrierungseinheit zugeführt werden, in der vorzugsweise destillativ unter Vakuum durch Abtrennung von nicht umgesetztem sek.-Butylbenzol und/oder Cumol der Gehalt an sek.-Butylbenzolhydroperoxid auf bis zu 65 Gew.-% und/oder Cumolhydroperoxid auf bis zu 10 Gew.-% erhöht wird. Abgetrenntes sek.-Butylbenzol und/oder Cumol wird vorzugsweise nach ggf. ergänzender Aufbereitung zum Oxidationsschritt zurückgeführt.

Der Stoffstrom aus der Aufkonzentrierung wird nun der Spaltung zugeführt, die erfindungsgemäß bevorzugt säurekatalysiert, vorzugsweise mittels Schwefelsäure, in homogener Phase erfolgt. Sek.-Butylbenzolhydroperoxid und Cumolhydroperoxid werden hier im wesentlichen zu Phenol, MEK und Aceton umgesetzt. Geeignete Spaltreaktoren und Reaktionsbedingungen können z. B. aus EP-0 589 588 A1 oder WO 97/06905 übernommen werden. Es kann vorteilhaft sein, das Spaltprodukt analog zum Verfahren in diesen Patentdokumenten einer Nachtemperung zur Verringerung des Gehalts an einigen weiteren Nebenprodukten zu unterziehen. Anschließend erfolgt eine vorzugsweise destillative Aufarbeitung des Spaltproduktgemisches auf dem Fachmann vertraute, zum Cumolverfahren analoge Weise, um die Wertprodukte Phenol, MEK und Aceton zu isolieren. Dabei fällt eine Acetophenonhaltige Schwersieder-Fraktion an, aus der das Acetophenon z. B. nach dem Verfahren gemäß US-4 559 110 oder durch thermisches Cracken der Schwersieder und destillative Aufbereitung isoliert werden kann. Acetophenon kann so ebenfalls als den Rückstand verminderndes Wertprodukt gewonnen werden.

Das erfindungsgemäße Verfahren ist nicht auf die hier skizzierten Hauptverfahrensschritte beschränkt; vielmehr können alle aus dem Cumolverfahren bekannten Verfahrensvarianten, Ergänzungen oder Verschaltungen auch auf ihre Eignung für das erfindungsgemäße Verfahren geprüft werden. Erfindungswesentlich bleibt dabei stets der Oxidationsschritt mit einem sek.-Butylbenzol und Cumol enthaltenden Einsatzgemisch mit einem Cumolgehalt von 3 bis 15 Gew.-%, um die Oxidationsgeschwindigkeit zu erhöhen und die Nebenproduktbildung zu verringern. Auf diese Weise lassen sich Spalt-Ausbeuten an den Wertprodukten Phenol, MEK und Aceton von über 95 % bezogen auf die Einsatzstoffe sek.-Butylbenzol und Cumol erzielen.

Das erfindungsgemäße Verfahren wird durch die folgenden Beispiele näher erläutert, ohne darauf beschränkt zu sein:

### Vergleichsbeispiel 1:

Ein Gemisch enthaltend 85 Gew.-% sek.-Butylbenzol und 15 Gew.-% Cumolhydroperoxid wird mit Sauerstoff in einem thermostatisierbaren Blasensäulenreaktor oxidiert. Die Reaktionstemperatur beträgt 132 °C. Nach 30, 90 und 120 Minuten werden dem Reaktor Proben entnommen und auf ihren Gehalt an sek.-Butylbenzolhydroperoxid (kurz: BHP) und Cumolhydroperoxid (kurz: CHP) sowie an einigen Nebenprodukten untersucht. Die Ergebnisse zeigt Tabelle 1 (Carbinol steht für Dimethylphenylcarbinol).

**Tabelle 1**

| Zeit Min. | BHP Gew.-% | CHP Gew.-% | Acetophenon Gew.-% | Propiophenon Gew.-% | Carbinol Gew.-% | 2-Phenylbutanol Gew.-% |
|---|---|---|---|---|---|---|
| 30 | 5,15 | 14,04 | 0,37 | 0,01 | 1,48 | 0,04 |
| 90 | 14,79 | 13,32 | 1,61 | 0,1 | 2,74 | 0,32 |
| 120 | 18,61 | 13,15 | 2,37 | 0,15 | 3,30 | 0,61 |

### Vergleichsbeispiel 2:

Unter den gleichen Bedingungen wie in Vergleichsbeispiel 1 wird ein Gemisch aus sek.-Butylbenzol mit 50 Gew.-% Cumol bezogen auf den sek.-Butylbenzolgehalt oxidiert. Die Probenahme erfolgt nach 150 und 180 Minuten. Die Ergebnisse zeigt Tabelle 2.

**Tabelle 2**

| Zeit Min. | BHP Gew.-% | CHP Gew.-% | Acetophenon Gew.-% | Propiophenon Gew.-% | Carbinol Gew.-% | 2-Phenylbutanol Gew.-% |
|---|---|---|---|---|---|---|
| 150 | 6,6 | 10,46 | 0,95 | 0,06 | 0,85 | 0,16 |
| 180 | 8,96 | 13,66 | 1,51 | 0,09 | 1,42 | 0,27 |

### Beispiel 3 (erfindungsgemäß):

Unter den gleichen Bedingungen wie in Vergleichsbeispiel 1 wird ein Gemisch aus 95 Gew.-% sek.-Butylbenzol und 5 Gew.-% Cumol oxidiert. Nach 270, 300 und 330 Minuten wird der Gehalt der in Vergleichsbeispiel 1 geprüften Stoffe im Oxidat ermittelt. Die Ergebnisse zeigt Tabelle 3.

**Tabelle 3**

| Zeit Min. | BHP Gew.-% | CHP Gew.-% | Acetophenon Gew.-% | Propiophenon Gew.-% | Carbinol Gew.-% | 2-Phenylbutanol Gew.-% |
|---|---|---|---|---|---|---|
| 270 | 14,9 | 1,3 | 2,06 | 0,49 | 0,16 | 0,49 |
| 300 | 17,4 | 1,43 | 2,67 | 0,17 | 0,20 | 0,60 |
| 330 | 19,9 | 1,58 | 3,39 | 0,20 | 0,26 | 0,86 |

Verglichen mit den Vergleichsbeispielen 1 und 2 ist trotz der wesentlich längeren Oxidationszeit der Gehalt an Carbinol im Oxidat deutlich reduziert, während die Acetophenonbildung nur mäßig stärker ausfällt. Die erfindungsgemäße Oxidation wirkt sich daher positiv auf die Ruckstandsbildung aus, da das Acetophenon als Wertstoff gewonnen werden kann. Im Vergleich zu Vergleichsbeispiel 1 und 2 ist der summarische Anteil der Nebenprodukte 2-Phenylbutanol, Carbinol und Propiophenon bei vergleichbaren BHP-Gehalten deutlich niedriger.

### Beispiel 4 (erfindungsgemäß):

Unter den gleichen Bedingungen wie im Vergleichsbeispiel 1 wird ein Gemisch aus 90 Gew.-% sek.-Butylbenzol und 10 Gew.-% Cumol oxidiert. Nach 300 und 330 Minuten wird der Gehalt der in Vergleichsbeispiel 1 untersuchten Stoffe im Oxidat ermittelt. Die Ergebnisse zeigt Tabelle 4.

**Tabelle 4**

| Zeit Min. | BHP Gew.-% | CHP Gew.-% | Acetophenon Gew.-% | Propiophenon Gew.-% | Carbinol Gew.-% | 2-Phenylbutanol Gew.-% |
|---|---|---|---|---|---|---|
| 300 | 19,3 | 3,33 | 3,18 | 0,18 | 0,52 | 0,80 |
| 330 | 22,20 | 3,67 | 4,18 | 0,25 | 0,68 | 1,08 |

Verglichen mit den Vergleichsbeispielen 1 und 2 ist trotz der wesentlich längeren Oxidationszeit der Gehalt an Carbinolen im Oxidat deutlich geringer. Wird Acetophenon als Wertstoff isoliert, wird die Rückstandmenge damit nachhaltig verringert.

### Vergleichsbeispiel 5:

Unter den gleichen Bedingungen wie in den Beispielen 3 und 4 wird reines sek.-Butylbenzol oxidiert. Nach 330 Minuten werden die in Tabelle 5 dargestellten Werte ermittelt.

**TABELLE 5**

| Zeit Min. | BHP Gew.-% | Acetophenon Gew.-% | Propiophenon Gew.-% | 2-Phenylbutanol Gew.-% |
|---|---|---|---|---|
| 330 | 11,3 | 1,62 | 0,11 | 0,40 |

Durch den in Vergleich zu den Beispielen 3 und 4 deutlich geringeren sek.-Butylbenzolhydroperoxidgehalt von nur etwa 11 Gew.-% im Oxidat wird die hohe Oxidationsgeschwindigkeit beim erfindungsgemäßen Verfahren klar belegt.

### Beispiel 6 (erfindungsgemaß):

Ein Gemisch, bestehend aus 95 % sek.-Butylbenzol und 5 % Cumol wird bei 132 °C mit Sauerstoff in einem thermostatisierbaren Blasensäulenreaktor oxidiert. Nach einer Oxidationszeit von 5,5 Stunden enthält das Oxidat laut GC-Analyse 1,14 Gew.-% CHP und 18,5 Gew.-% sek.-Butylbenzolhydroperoxid. Dieses Gemisch wird anschließend im Hochvakuum einer Konzentrierung unterworfen, wobei das anfallende Konzentrat laut GC-Analyse 3,49 Gew.-% CHP und 58,18 Gew.-% sek.-Butylbenzolhydroperoxid enthält. Das als Destillat anfallende Kohlenwasserstoffgemisch enthält weniger als 1 Gew.-% Peroxid, berechnet als CHP. Das Konzentrat wird anschließend bei 50 °C einer einphasigen Spaltung in Gegenwart von 2000 ppm Schwefelsäure, gelöst in Aceton oder in einem Testspaltprodukt bestehend aus Aceton, MEK, Phenol und den Kohlenwasserstoffen gespalten.

Die spaltausbeuten betragen nach Auswertung der GC-Analyse für Aceton über 95 %, für MEK über 95 % und für Phenol über 95 %.

## Patentansprüche

1. Verfahren zur Herstellung von Phenol, Methylethylketon und Aceton durch Oxidation eines sek.-Butylbenzol und Cumol enthaltenden Gemisches mit molekularen Sauerstoff enthaltendem Gas und Hocksche Spaltung von bei der Oxidation gebildetem sek. -Butylbenzol- und Cumolhydroperoxid,
dadurch gekennzeichnet,
daß der Cumolgehalt im sek.-Butylbenzol und Cumol enthaltenden Gemisch 3 bis 15 Gew.-% beträgt.

2. Verfahren nach Anspruch 1,
dadurch gekennzeichnet,
daß der Cumolgehalt im Gemisch 3 bis 10 Gew.-% beträgt.

3. Verfahren nach Anspruch 2,
dadurch gekennzeichnet,
daß der Cumolgehalt im Gemisch 5 bis 10 Gew.-% beträgt.

4. Verfahren nach zumindest einem der vorherigen Ansprüche,
dadurch gekennzeichnet,
daß das Gemisch aus sek.-Butylbenzol und Cumol jeweils hoher Reinheit besteht.

5. Verfahren nach zumindest einem der vorherigen Ansprüche,
dadurch gekennzeichnet,
daß die Oxidation bei Temperaturen von 100 °C bis 140 °C und Drücken von 1 bis 20 bar absolut erfolgt.

6. Verfahren nach zumindest einem der vorherigen Ansprüche,
dadurch gekennzeichnet,
daß als molekularen Sauerstoff enthaltendes Gas Luft dient.

7. Verfahren nach zumindest einem der vorherigen Ansprüche,
dadurch gekennzeichnet,
daß das Oxidat vor der Spaltung destillativ durch Abtrennung von sek.-Butylbenzol oder Cumol oder beidem aufkonzentriert wird.

8. Verfahren nach Anspruch 7,
dadurch gekennzeichnet,
daß abgetrenntes sek.-Butylbenzol oder Cumol oder beides wieder der Oxidation zugeführt wird.

9. Verfahren nach zumindest einem der vorherigen Ansprüche,
dadurch gekennzeichnet,
daß das Spaltgemisch homogen ist.

10. Verfahren nach zumindest einem der vorherigen Ansprüche,
dadurch gekennzeichnet,
daß die Spaltung säurekatalysiert wird.

11. Verfahren nach Anspruch 10,
dadurch gekennzeichnet,
daß als Säurekatalysator Schwefelsaure dient.

12. Verfahren nach zumindest einem der vorherigen Ansprüche,
dadurch gekennzeichnet,
daß Acetophenon aus dem Spaltproduktgemisch isoliert wird.
